Europäisches Patentamt

European Patent Office

Office européen des brevets

(19)

(11) Veröffentlichungsnummer: **0 020 961**
B1

(12) **EUROPÄISCHE PATENTSCHRIFT**

(45) Veröffentlichungstag der Patentschrift:
**12.05.82**

(51) Int. Cl.³: **C 12 M 1/28**

(21) Anmeldenummer: **80102505.7**

(22) Anmeldetag: **08.05.80**

(54) Vorrichtung zum Nachweis von Mikroorganismen.

(30) Priorität: **20.06.79 CH 5759/79**

(43) Veröffentlichungstag der Anmeldung:
**07.01.81 Patentblatt 81/1**

(45) Bekanntmachung des Hinweises auf die Patenterteilung:
**12.05.82 Patentblatt 82/19**

(84) Benannte Vertragsstaaten:
**AT BE CH DE FR GB IT LI NL**

(56) Entgegenhaltungen:
**keine**

(73) Patentinhaber: **F. HOFFMANN-LA ROCHE & CO.
Aktiengesellschaft, CH-4002 Basel (CH)**

(72) Erfinder: **Kloter, Rudolf, Waldhofstrasse 5,
CH-4310 Rheinfelden (CH)**

(74) Vertreter: **Kloter, Rudolf et al,
Grenzacherstrasse 124 Postfach 601, CH-4002 Basel
(CH)**

## Vorrichtung zum Nachweis von Mikroorganismen

Die vorliegende Erfindung betrifft eine Vorrichtung zum Nachweis von Mikroorganismen, z. B. in Körperflüssigkeiten.

Zum Nachweis von Mikroorganismen in Körperflüssigkeiten, insbesondere von Bakterien im Blut, ist es notwendig, flüssiges Nährmedium zu beimpfen und anschließend das Wachstum auf einem festen Nährboden weiterzuführen. Seit Jahren gibt es Systeme, bei denen beide Nährmedien im gleichen Behälter kombiniert sind, um die lästige und unter Umständen riskante Überführung der Vorkulturen im flüssigen Nährmedium auf einen festen Nährboden außerhalb des Behälters zu vermeiden.

Die meisten bekannten Vorrichtungen, in welchen ein festes und ein flüssiges Nährmedium kombiniert werden, sind jedoch aus vielen Gründen nicht zufriedenstellend. Insbesondere kann wegen der Gefahr des Herauslösens von Bestandteilen des festen Nährbodens in die flüssige Nährbouillon meistens nur ein mit der flüssigen Nährbouillon kompatibles festes Nährmedium verwendet werden. dies hat unter anderem den erheblichen Nachteil, daß keine Differenzierung der Erreger möglich ist.

Die bisher vorgeschlagenen Lösungen zur Separierung des festen Nährbodens und der flüssigen Nährbouillon während der Inkubationszeit und des Transportes sind aufwendig und kostpielig und/oder gewährleisten eine Separierung beider Medien nur während der Inkubation, jedoch nicht während des Transportes.

In der DE-A-2 806 902 wird ein System vorgeschlagen, das die genannten Nachteile nicht aufweist. Dieses System besteht aus einem ersten Behälter enthaltend ein flüssiges Nährmedium und einem zweiten Behälter enthaltend ein festes Nährmedium, wobei die Innenräume beider Behälter in Kontakt sind und diese Behälter so verbunden sind, daß sie wieder getrennt werden können.

Fig. 3 dieser Druckschrift zeigt die Kombination der beiden Behälter. Die auf diese Weise gebildete Vorrichtung enthaltend mindestens ein festes und ein flüssiges Nährmedium wird mehrmals gekippt, um einen optimalen Kontakt zwischen beiden Nährmedien zu gewährleisten. Nach Bebrütung bei 20−37°C während 1 Stunde bis 10 Tagen wird das auf dem festen Nährboden allfällig vorhandene Wachstum beobachtet und bewertet. Ist kein Wachstum feststellbar, kann dieser Vorgang mehrmals wiederholt werden. Bei längerer Inkubation wird die Vorrichtung mindestens täglich gekippt, um einen optimalen Kontakt zwischen dem flüssigen und dem festen Nährmedium zu gewährleisten.

Dieser Figur kann man entnehmen, daß der Behälter enthaltend das feste Nährmedium mit einem Schraubverschluß mit dem Behälter enthaltend das flüssige Nährmedium verbunden ist. Zur Entfernung des Behälters, enthaltend das feste Nährmedium (z. B. für den anschließenden Transport in das Laboratorium zur Bestimmung der Keime auf dem festen Nährboden), muß dieser Schraubverschluß gelöst werden. Dabei könnte die Gefahr bestehen, daß im oberen Behälter noch vorhandenes flüssiges Nährmedium (als Folge des vorerwähnten Kippens) an der Glaswand hinunterläuft und das Gewinde benetzt. Dies könnte zur Folge haben, daß die Person, welche die Behälter trennt, mit diesem flüssigen Nährmedium in Kontakt kommen könnte, was auf alle Fälle zu vermeiden ist.

Ein weiterer kritischer Punkt der Vorrichtung nach der DE-A-2 806 902 ist das in Fig. 3 gezeigte Innengewinde 12 des oberen Behälters. Bekanntlich sind ja Innengewinde viel aufwendiger in der Herstellung als Außengewinde.

Aufgabe der vorliegenden Erfindung war, eine Vorrichtung zur Bestimmung von Mikroorganismen zu schaffen, welche in jeder Beziehung optimal ist.

Die vorliegende Erfindung betrifft demnach eine Vorrichtung zum Nachweis von Mikroorganismen, bestehend aus einem ersten Behälter mit Verschlußvorrichtung für ein flüssiges Nährmedium und einem Transportbehälter mit Verschlußvorrichtung und daran befestigtem Träger für mindestens ein festes Nährmedium. Die Erfindung besteht bei einer derartigen Vorrichtung im wesentlichen darin, daß die Verschlußvorrichtungen des ersten Behälters und des Transportbehälters austauschbar sind.

Weitere Einzelheiten und Merkmale der vorliegenden Erfindung ergeben sich aus der nachstehenden detaillierten Beschreibung einer Ausführungsform der erfindungsgemäßen Vorrichtung.

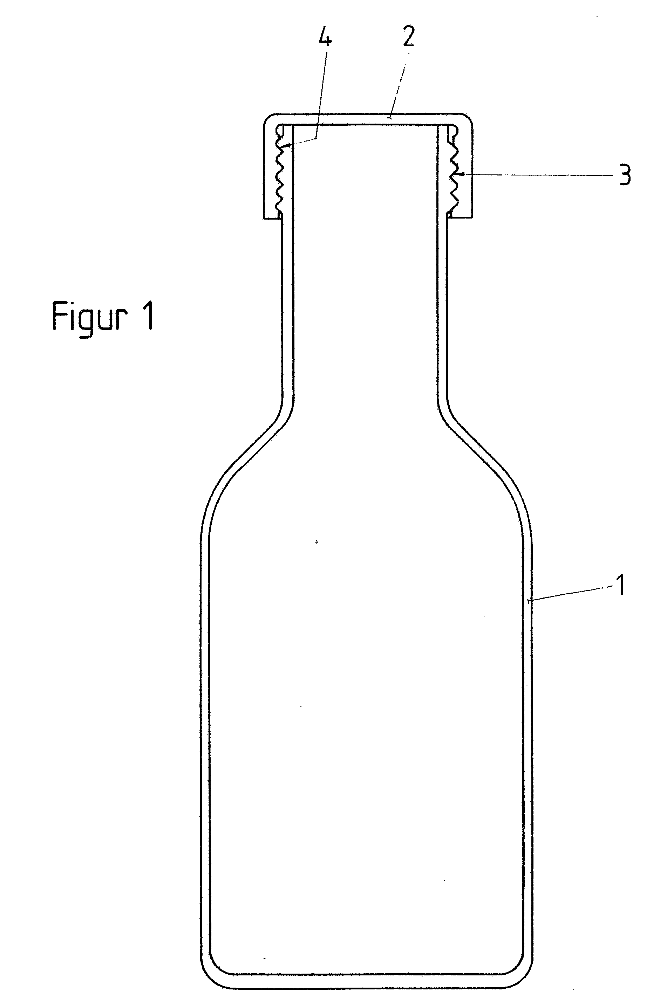

Fig. 1 zeigt schematisch den ersten Behälter mit Verschlußvorrichtung für ein flüssiges Nährmedium;

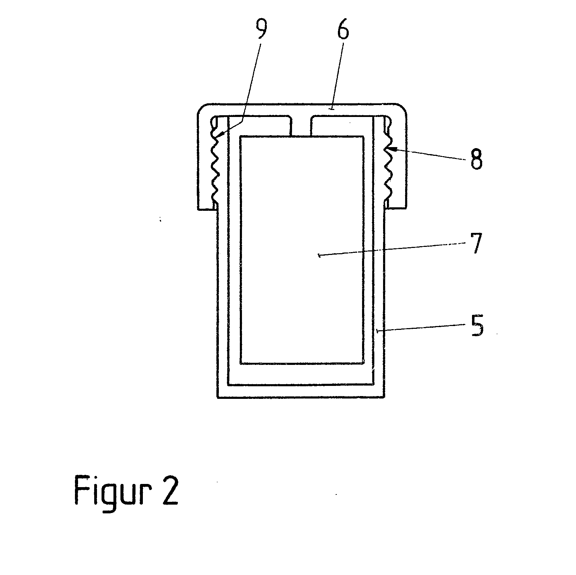

Fig. 2 zeigt schematisch den Transportbehälter mit Verschlußvorrichtung und daran befestigtem Träger für mindestens ein festes Nährmedium;

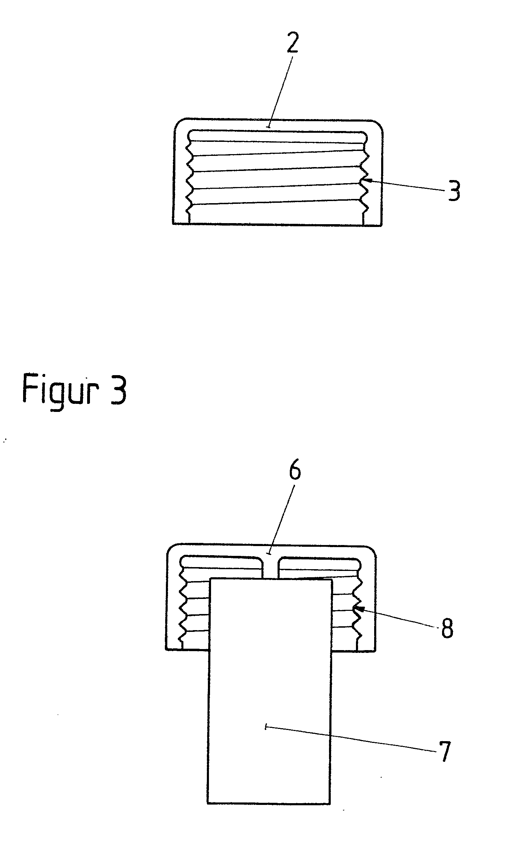

Fig. 3 zeigt schematisch die beiden Verschlußvorrichtungen;

Fig. 4 zeigt schematisch die Kombination des Behälters von Fig. 1 mit der Verschlußvorrichtung und daran befestigtem Träger des Transportbehälters von Fig. 2.

Der erste Behälter für das flüssige Nährmedium besteht aus einer Flasche 1, die vorzugsweise aus einem transparenten Material wie Glas oder Kunststoff besteht. Die Flasche wird durch einen Schraubdeckel 2 verschlossen. Der Schraubdeckel zeigt das Innengewinde 3. Demgegenüber hat der Behälter ein Außengewinde 4. Der Schraubdeckel ist mit einer Vorrichtung versehen, die es erlaubt, eine Körperflüssigkeit, wie Blut, mit einer Nadel in die Flasche einzuführen (was in der Figur nicht gezeigt ist). Der Transportbehälter 5 besteht aus einem durchsichtigen Rohr aus Glas oder Kunststoff.

Am Schraubdeckel 6 des Transportbehälters ist ein Träger 7, z. B. ein Objektträger befestigt, welcher für mindestens ein festes Nährmedium vorgesehen ist. Der Schraubdeckel 6 hat ein Innengewinde 8. Der Transportbehälter 5 hat demgegenüber ein Außengewinde 9.

Es ist zu beachten, daß die Innengewinde 3 und 8 sowie die Außengewinde 4 und 9 identisch sind.

Bei Gebrauch wird mit Hilfe eines mit einer Nadel versehenen Übertragungsgerätes Blut von einem Patienten in den Behälter 1 geführt. Der Behälter, welcher mit flüssigem Nährmedium 10 versehen ist, steht unter leichtem Vakuum, damit das Fließen des Blutes in den Behälter erleichtert wird.

Wenn der Behälter die gewünschte Menge an Blut enthält, wird während etwa 1 Stunde bis zu etwa 10 Tagen bei 20−37°C inkubiert.

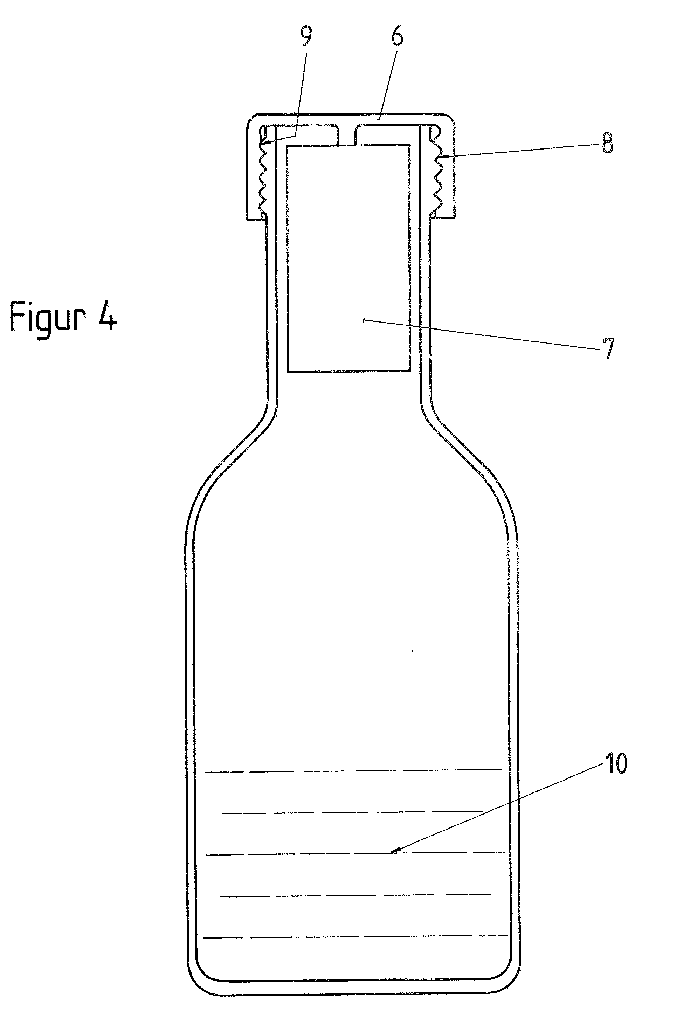

Anschließend werden die Schraubdeckel 2 und 6 gegeneinander vertauscht. Dadurch entsteht die in Fig. 4 abgebildete Vorrichtung. Die auf diese Weise gebildete Vorrichtung, enthaltend mindestens ein festes und ein flüssiges Nährmedium, wird mehrmals gekippt, um einen optimalen Kontakt zwischen beiden Nährmedien zu gewährleisten. Nach Bebrütung bei 20−37°C während 1 Stunde bis 10 Tagen wird das auf dem festen Nährboden allfällig vorhandene Wachstum beobachtet. Zur Auswertung des Wachstums auf dem festen Nährmedium werden die Schraubdeckel 2 und 6 erneut gegeneinander vertauscht. Der Transportbehälter mit dem festen Nährmedium kann dann problemlos in das Laboratorium zur weiteren Untersuchung geschickt werden.

Die vorliegende Erfindung ist selbstverständlich nicht auf die bereits beschriebene spezifische Ausführungsform beschränkt. So können z. B. im Rahmen der vorliegenden Erfindung die beiden Behälter 1 und 5 eine beliebige Form besitzen und auf verschiedene Weise verschlossen sein. Wichtig ist indessen, daß bei der Vorrichtung gemäß Fig. 4 das feste Nährmedium nicht in das flüssige Nährmedium 10 hineinragt. Erfindungswesentlich ist, daß die Verschlußvorrichtungen vom Behälter für flüssiges Nährmedium und für Transportbehälter austauschbar sind.

Zum Verschließen der Behälter brauchen natürlich nicht unbedingt Schraubverschlüsse verwendet werden. Das Verbinden der beiden Behälter kann auch z. B. durch Bajonettverschlüsse erfolgen.

## Patentansprüche

1. Vorrichtung zum Nachweis von Mikroorganismen, bestehend aus einem ersten Behälter (1) mit Verschlußvorrichtung (2) für ein flüssiges Nährmedium (10) und einem Transportbehälter (5) mit Verschlußvorrichtung (6) und daran befestigtem Träger (7) für mindestens ein festes Nährmedium, dadurch gekennzeichnet, daß die Verschlußvorrichtungen (2 bzw. 6) des ersten Behälters (1) und des Transportbehälters (5) austauschbar sind.

2. Vorrichtung nach Anspruch 1, dadurch gekennzeichnet, daß die Verschlußvorrichtungen (2 bzw. 6) Schraubverschlüsse sind.

3. Vorrichtung nach Anspruch 1, dadurch gekennzeichnet, daß die Verschlußvorrichtungen (2 bzw. 6) Bajonettverschlüsse sind.

## Claims

1. Apparatus for the detection of microorganisms, consisting of a first container (1) with closure means (2) for a liquid nutrient medium (10) and a transport container (5) with closure means (6) and carrier (7) attached thereto for at least one solid nutrient medium, characterized in that the closure means (2 or 6) of the first container (1) and of the transport container (5) are interchangeable.

2. Apparatus according to claim 1, characterized in that the closure means (2 or 6) are screw closures.

3. Apparatus according to claim 1, characterized in that the closure means (2 or 6) are bayonet closures.

## Revendications

1. Dispositif pour déceler la présence de microorganismes, consistant en un premier récipient (1) avec dispositif de fermeture (2) pour un milieu nutritif liquide (10) et un récipient de transport (5) avec dispositif de fermeture (6) et, fixé sur ce dernier, un support (7) pour au moins un milieu nutritif solide, caractérisé en ce que les dispositifs de fermeture (2 et 6 respectivement) du premier récipient (1) et du récipient de transport (5) sont échangeables.

2. Dispositif selon la revendication 1, caractérisé en ce que les dispositifs de fermeture (2 et 6 respectivement) sont des fermetures à vis.

3. Dispositif selon la revendication 1, caractérisé en ce que les dispositifs de fermeture (2 et 6 respectivement) sont des fermetures à baïonnettes.

Figur 1

Figur 2

2

3

Figur 3

6

8

7

Figur 4